# EUROPEAN PATENT APPLICATION

(11) **EP 1 849 768 A1**
(43) Date of publication of application: **31.10.2007**
(21) Application number: 06713077.3
(22) Date of filing: 31.01.2006
(51) Int. Cl.: C07C 319/06, C07C 323/20

(54) **METHODS FOR PRODUCING 2,6-DI-TERT-BUTYL-4-MERCAPTOPHENOL AND 4,4'-ISOPROPYLIDENEDITHIOBIS[2,6-DI-TERT-BUTYLPHENOL]**

(30) Priority: 04.02.2005 JP 2005029627
(71) Applicant: Sumitomo Chemical Company, Limited, Tokyo 104-8260 (JP)
(72) Inventor: YOKOYAMA, Hiroaki, Hyogo 6600054 (JP); YAMAMOTO, Tomiaki, Gifu, 5008082 (JP); INOUE, Haruyuki, Gifu 5010521 (JP)
(74) Representative: Winter, Brandl, Fürniss, Hübner Röss, Kaiser, Polte Partnerschaft Patent- und Rechtsanwaltskanzlei
(86) International application number: PCT/JP2006/301934
(87) International publication number: WO 2006/082950

(57) **Abstract**

The present invention provides a method for producing 2,6-di-tert-butyl-4-mercaptophenol containing reducing bis(3,5-di-tert-butyl-4-hydroxyphenyl) polysulfide with zinc in a mixed solvent of toluene and n-butanol, and provides a method for producing 4,4'-isopropylidenedithiobis[2,6-di-tert-butylphenol] using the above-mentioned method.

## Description

### Technical Field

The present invention relates to 4,4'-isopropylidenedithiobis[2,6-di-tert-butylphenol] as an antihyperlipidemic drug and its intermediate 2,6-di-tert-butyl-4-mercaptophenol.

### Background Technology

2,6-di-tert-butyl-4-mercaptophenol is a useful intermediate of 4,4'-isopropylidenedithiobis[2,6-di-tert-butylphenol] as an antihyperlipidemic drug, and as the production method thereof, there is conventionally suggested a method of reducing bis(3,5-di-tert-butyl-4-hydroxyphenyl) polysulfide using zinc in toluene (see, USP 3479407), however, the reaction is not consistent due to stoppage of the reducing reaction on the way, and the like, thus, it is necessary to perform the reaction for a long period of time using zinc and an acid in large excess amount. There is suggested, for solving the problem of this method, a method of carrying out reduction with zinc in a mixed solvent of toluene and ethanol (see, JP 2004-107294-A). In this method, however, zinc tends to sink in the reaction system to increase stirring load, leading to a problem of poor stirring efficiency. Thus, the charging amount of zinc is limited, and a special stirring apparatus of large torque is necessary. Further, in this method, reaction temperatures of 55 to 60°C are necessary, leading to needs of heating and cooling after completion of the reaction, however, if industrial production is taken into consideration, it is desirable that the reaction can be controlled without performing such heating and cooling.

### Disclosure of the Invention

The object of the present invention is to provide methods for producing 2,6-di-tert-butyl-4-mercaptophenol and 4,4'-isopropylidenedithiobis[2,6-di-tert-butylphenol] efficiently and industrially advantageously.

The present invention is as described below.
<1> A method for producing 2,6-di-tert-butyl-4-mercaptophenol containing reducing bis(3,5-di-tert-butyl-4-hydroxyphenyl) polysulfide with zinc in a mixed solvent of toluene and n-butanol.
<2> The method according to <1> wherein the reduction is carried out without heating.
<3> The method according to <1> or <2> wherein the reduction is carried out at a temperature in the range of 15 to 35°C.
<4> The method according to any one of <1> to <3> wherein the volume ratio of n-butanol to toluene is 5/100 to 30/100.
<5> A method for producing 2,6-di-tert-butyl-4-mercaptophenol containing a process of reacting 2,6-di-tert-butylphenol with sulfur monochloride in toluene to obtain bis(3,5-di-tert-butyl-4-hydroxyphenyl) polysulfide, and a process of reducing bis(3,5-di-tert-butyl-4-hydroxyphenyl) polysulfide with zinc in a mixed solvent of toluene and n-butanol.
<6> The method according to <5> wherein the reaction of 2,6-di-tert-butylphenol with sulfur monochloride is carried out at a temperature in the range of -10°C to -7°C.
<7> The method according to <5> or <6> wherein the reaction in the reduction process is carried out without heating.
<8> The method according to any one of <5> to <7> wherein the reaction in the reduction process is carried out at a temperature in the range of 15 to 35°C.
<9> The method according to any one of <5> to <8> wherein the volume ratio of n-butanol to toluene in the reduction process is 5/100 to 30/100.
<10> A method for producing 4,4'-isopropylidenedithiobis[2,6-di-tert-butylphenol] containing a process of reducing bis(3,5-di-tert-butyl-4-hydroxyphenyl) polysulfide with zinc in a mixed solvent of toluene and n-butanol to obtain 2,6-di-tert-butyl-4-mercaptophenol, and a process of reacting 2,6-di-tert-butyl-4-mercaptophenol with acetone under an acidic condition.
<11> The method according to <10> wherein the reaction in the reduction process is carried out without heating.
<12> The method according to <10> or <11> wherein the reaction in the reduction process is carried out at a temperature in the range of 15 to 35°C.
<13> The method according to any one of <10> to <12> wherein the volume ratio of n-butanol to toluene in the reduction process is 5/100 to 30/100.

### Best Modes for Carrying Out the Invention

The present invention will be illustrated in detail below. 1. Production of 2,6-di-tert-butyl-4-mercaptophenol

Bis(3,5-di-tert-butyl-4-hydroxyphenyl) polysulfide to be used in the present invention includes bis(3,5-di-tert-butyl-4-hydroxyphenyl) disulfide, bis(3,5-di-tert-butyl-4-hydroxyphenyl) trisulfide and bis(3,5-di-tert-butyl-4-hydroxyphenyl) tetrasulfide, and also, sulfides containing further larger number of -S-. The number of -S- is not restricted. Included are both a case of single use of these sulfides and a case of a mixture of two or more sulfides.

The method for producing 2,6-di-tert-butyl-4-mercaptophenol is a method containing reducing bis(3,5-di-tert-butyl-4-hydroxyphenyl) polysulfide (hereinafter, described as sulfide body in some cases) with zinc in a mixed solvent of toluene and n-butanol, and the reduction is carried out usually in the presence of hydrochloric acid.

The solvent in the present invention is a mixed solvent of toluene and n-butanol, and by use of this solvent, zinc is dispersed easily and stirring efficiency is improved remarkably.

Further, conventionally, reaction temperatures of 55 to 60°C are necessary, leading to needs of heating and cooling before post-treatment, however, by use of a mixed solvent of toluene and n-butanol, the reaction progresses smoothly around room temperature (at 15 to 35°C), thus, needs of heating and cooling thereafter disappear and production cost can be reduced, and time required for charging and discharging of heat can be saved, indicating that this is an industrially advantageous method.

The amounts of toluene and n-butanol may advantageously be those giving stirring capability of the reaction system. The amounts of constituent components of the solvent are as described below.

The amount of toluene is usually 50 to 110 parts by weight, preferably 70 to 95 parts by weight per 100 parts by weight of bis(3,5-di-tert-butyl-4-hydroxyphenyl) polysulfide from the standpoint of easiness of stirring and economy.

The amount of n-butanol is usually 5 to 30 parts by volume, preferably 10 to 20 parts by volume per 100 parts by volume of toluene from the standpoint of reactivity and liquid separating property of the reaction liquid.

From the standpoint of volume efficiency, the concentration of hydrochloric acid is preferably 10% (w/w) or more, and concentrated hydrochloric acid of 35% (w/w) is particularly preferable.

The amount of hydrochloric acid is usually 4 to 6 mol, preferably 4.6 to 5.3 per mol of bis(3,5-di-tert-butyl-4-hydroxyphenyl) polysulfide. Since addition of hydrochloric acid is accompanied by heat generation, the addition is usually carried out by dropping.

The amount of zinc is usually 1.3 to 2.1 mol, preferably 1.6 to 1.8 mol per mol of bis(3,5-di-tert-butyl-4-hydroxyphenyl) polysulfide from the standpoint of reaction rate and economy. Zinc of any configuration may be used, and for example, zinc powders which are usually commercially sold are mentioned.

The reaction temperature is around room temperature, and specifically, usually in the range of 15 to 35°C, more preferably in the range of 20 to 30°C from the standpoint of reaction speed and reaction control.

Addition of hydrochloric acid is accompanied by heat generation as described above, however, if necessary, cold insulation, heat insulation and regulation of dropping time may be advantageously conducted to control temperature in the above-mentioned range.

After completion of dropping of hydrochloric acid, for example, termination can be made when the peak area of a sulfide body reaches 1% or lower by a high performance liquid chromatography (HPLC) method, and usually, the reaction ends in 1 to 2 hours.

After completion of the reaction, 2,6-di-tert-butyl-4-mercaptophenol can be isolated and purified easily, for example, by a method described below.

After completion of the reaction, if insoluble materials are present, these are filtrated off, and an acid such as hydrochloric acid and the like (preferable concentration of hydrochloric acid is about 10% (w/w)) is added to the filtrate and the liquid is washed, and allowed to stand still to cause liquid separation. The organic layer is washed with water, then, extracted using an alkali, and this is acidified, to allow deposition of 2,6-di-tert-butyl-4-mercaptophenol in the form of crystal. The deposited crystal is filtrated, and this is washed with water and 50% (v/v) water-methanol, thus, 2,6-di-tert-butyl-4-mercaptophenol can be isolated and purified.

The above-mentioned alkali includes alkali metal hydroxides such as potassium hydroxide, sodium hydroxide, lithium hydroxide, and the like, and of them, potassium hydroxide is preferable. The alkali metal hydroxide is usually used in the form of aqueous solution, and its concentration is usually 5 to 10% (w/w), preferably 7 to 9% (w/w). It is preferable to deaerate the alkali aqueous solution previously for preventing oxidation of 2,6-di-tert-butyl-4-mercaptophenol. As the deaeration method, there are mentioned a method in which an operation of, after pressure reduction, recovering pressure with nitrogen is repeated several times, and other methods.

It is preferable that extraction using an alkali is carried out twice. For example, a first extraction is carried out using an alkali in an amount of usually 2.3 to 3.2 mol, preferably 2.3 to 2.5 mol based on 1 mol of bis(3,5-di-tert-butyl-4-hydroxyphenyl) polysulfide, then, a second extraction is further carried out using an alkali in an amount of usually 0.6 to 1.7 mol, preferably 0.6 to 0.8 mol. The temperature in extraction is usually 20 to 40°C , preferably 25 to 35°C.

Acidification of an alkali layer can be carried out using an acid, for example, concentrated hydrochloric acid (preferably, 35% (w/w)), and the temperature in acidification is usually 20 to 40°C , preferably 25 to 35°C. pH after acidification is 0.5 to 2.5, preferably 1 to 2.

### 2. Production of bis(3,5-di-tert-butyl-4-hydroxyphenyl) polysulfide

Bis(3,5-di-tert-butyl-4-hydroxyphenyl) polysulfide can be obtained by, for example, a method described in USP 3479407 in which 2,6-di-tert-butylphenol is reacted with sulfur monochloride in toluene. USP 3479407 describes that the reaction temperature is required to be controlled in -20 to -15°C and yield lowers at -7 to 0°C (see, the same publication, table 1).

The present inventors have found that the reaction can be carried out at high yield even in the case of performing the reaction in the range of -10 to -7°C.

Specifically, it is a method in which 2,6-di-tert-butylphenol is reacted with sulfur monochloride in the presence of catalytic amount of iodine. The reaction is preferably carried out in a solvent. Examples of the solvent include aliphatic or aromatic hydrocarbons (e.g., hexane, toluene, benzene and the like), halogenated hydrocarbons (e. g. , carbon tetrachloride, chlorobenzene and the like), alcohols (e.g., methanol and the like), ethers (e.g., diethyl ether and the like), ester solvents (e.g., ethyl acetate and the like), and the like, and preferable is toluene. The solvent may be used advantageously in amount giving stirring capability of the reaction system, and the amount thereof is usually 70 to 120 parts by weight, preferably 80 to 100 parts by weight based on 100 parts by weight of 2,6-di-tert-butylphenol.

The amount of sulfur monochloride is usually 0.45 to 0.6 mol, preferably 0.48 to 0.52 mol based on 1 mol of 2,6-di-tert-butylphenol.

The amount of iodine is usually 0.0001 to 1 mol, preferably 0.0002 to 0.5 mol based on 1 mol of 2,6-di-tert-butylphenol.

The reaction temperature is preferably in the range of -10 to -7°C as described above, and usually, the reaction is carried out at normal pressure or higher pressure.

After completion of the reaction, the mixture is washed with water, and the organic layer is concentrated, thus, bis(3,5-di-tert-butyl-4-hydroxyphenyl) polysulfide can be isolated. In the case of use of toluene as a solvent, the toluene solvent can be subjected to the subsequent process without concentration by adding necessary amount of n-butanol.

### 3. Production of 4,4'-isopropylidenedithiobis[2,6-di-tert-butylphenol]

2,6-di-tert-butyl-4-mercaptophenol can be converted into 4,4'-isopropylidenedithiobis[2,6-di-tert-butylphenol] according to, for example, a method described in USP 3576883. Specifically, 4,4'-isopropylidenedithiobis[2,6-di-tert-butylphenol] can be obtained by reacting with acetone under an acidic condition. The reaction is usually conducted in a solvent, and examples of the solvent include acetone, methanol, ethanol, isopropanol and the like. The solvent may be used advantageously in an amount giving stirring capability, and the use amount is usually 150 to 200 parts by weight, preferably 160 to 180 parts by weight based on 100 parts by weight of 2,6-di-tert-butyl-4-mercaptophenol.

The reaction is carried out under a condition acidified using an acid such as, for example, hydrochloric acid, sulfuric acid, phosphoric acid, methanesulfonic acid and the like, preferably hydrochloric acid. The amount of the acid is preferably 0.03 to 0. 3 mol, more preferably 0. 05 to 0. 2 mol based on 1 mol of 2,6-di-tert-butyl-4-mercaptophenol. The amount of acetone is usually 1.1 to 2 mol, preferably 1.2 to 1.4 mol based on 1 mol of 2,6-di-tert-butyl-4-mercaptophenol.

The reaction is carried out usually in the range of 40 to 70°C, preferably in the range of 48 to 58°C, usually for 1 to 10 hours.

After completion of the reaction, 4,4'-isopropylidenedithiobis[2,6-di-tert-butylphenol] contained in the mixture can be isolated and purified by an ordinary method. For example, the mixture after completion of the reaction is cooled to 0 to 5°C and the deposited crystal is filtrated, thus, 4,4'-isopropylidenedithiobis[2,6-di-tert-butylphenol] can be isolated. Further, the isolated 4,4'-isopropylidenedithiobis[2,6-di-tert-butylphenol] crystal can also be purified by re-crystallizing from isopropanol of 1.6 to 2-fold volume.

The present invention will be illustrated more specifically by examples below, but the present invention is no limited to them.

HPLC analysis was carried out under the following conditions.
Column: ODS column (YMC-Pack AM-302)
Mobile Phase:
   Liquid A: 0.1% (v/v) trifluoroacetic acid aqueous solution
   Liquid B: acetonitrile
   Liquid A : Liquid B = 8:92
Detection wavelength: UV 240 nm

### Example 1

To toluene (600 ml) was added 2,6-di-tert-butylphenol (600 g) and iodine (295 mg), and the mixture was cooled to -10°C. Under nitrogen flow, sulfur monochloride (31.4 g) was dropped at -7 to -10°C, and the resulting mixture was stirred for 0.5 hours. Then, sulfur monochloride (31.4 g) was dropped at the same temperature, the mixture was stirred for 0.5 hours at the same temperature, further, sulfur monochloride (141.4 g) was dropped, and the mixture was stirred for 1 hour at the same temperature. The fact that the amount of raw materials reached 0.05% was confirmed by the HPLC method, and water (126 ml) was added and the mixture was stirred at about 60°C, and allowed to stand still to cause liquid separation. The resultant toluene layer was divided into three portions.

To 1/3 (405 g) of the above-mentioned toluene layer was added n-butanol (25 ml) and a zinc powder (50 g), and 35% hydrochloric acid (235.5 g) was dropped into the mixture at 20 to 30°C over 3 hours to 3 hours and 30 minutes. Further, the mixture was stirred for 1 hour at the same temperature, and the fact that the amount of the bis(3,5-di-tert-butyl-4-hydroxyphenyl) polysulfide body (sulfide body) reached 1% or less was confirmed by the HPLC method. Insoluble materials were filtrated, and washed with toluene (10 ml) . The filtrate was allowed to stand still, to cause liquid separation. To the organic layer was added water (150 ml) and 35% hydrochloric acid (50 ml) and the mixture was stirred, and allowed to stand still to cause separation. The organic layer was washed with water (67 ml) . At 25 to 35°C , the organic layer was extracted with deaerated 8% (w/w) potassium hydroxide aqueous solution (816 g). Further, extraction was performed with the same potassium hydroxide aqueous solution (270.7 g), and the extracted portion was combined with the previous alkali layer. To the resulting alkali layer was added 35% hydrochloric acid (163.7 g) to give pH 1 to 2, and the mixture was stirred for 30 minutes. The deposited crystal was filtrated, and washed with water (400 ml), then, with 50% (v/v) water-methanol (400 ml), and dried to obtain 2,6-di-tert-butyl-4-mercaptophenol (204.5 g). The yield was 88.5%, and the purity was 97%.

### Example 2

To 1/3 (405 g) of the toluene layer obtained in Example 1 was added 15 ml of n-butanol, and reduction with zinc was performed in the same manner as in Example 1. As a result, 2,6-di-tert-butyl-4-mercaptophenol (202 g) was obtained. The yield was 87.4%, and the purity was 97.8%.

### Example 3

To 1/3 (405 g) of the toluene layer obtained in Example 1 was added n-butanol (20 ml), and reduction with zinc was performed in the same manner as in Example 1. As a result, 2,6-di-tert-butyl-4-mercaptophenol (202 g) was obtained. The yield was 87.6%, and the purity was 97%.

### Example 4

To a toluene layer (406 g) of a sulfide body produced in the same method as in Example 1 was added n-butanol (20 ml), and reduction with zinc was carried out in the same manner as in Example 1. As a result, 2,6-di-tert-butyl-4-mercaptophenol (203.5 g) was obtained. The yield was 88%, and the purity was 98.4%.

### Example 5

To 2,6-di-tert-butyl-4-mercaptophenol (100 g) produced in Example 4 was added methanol (170 ml) and water (10 g) and the mixture was heated at 50 to 55°C. Acetone (29.2 g) was added to this, and 35% hydrochloric acid (4 g) was dropped into the mixture which was reacted for 5 hours at the same temperature. The fact that the amount of raw materials reached 5% or less was confirmed by the HPLC method, then, the mixture was cooled to 0 to 5°C , and aged for about 3 hours at the same temperature. The mixture was filtrated, and the crystal was washed with 80% isopropanol (25.7 ml).

The wet crystal (123.3 g) was dissolved in 218 ml of isopropanol with heating, and the solution was filtrated, then, the filtration apparatus and the like used were washed with isopropanol (17.3 ml). The filtrate and washing liquid were combined, and once heated and complete dissolution thereof was confirmed, then, the solution was cooled and aged at 0 to 5°C. The crystal was filtrated, washed with isopropanol (30 ml), and dried to obtain a crystal (88.74 g) of 4,4'-isopropylidenedithiobis[2,6-di-tert-butylphenol]. The yield of 82% and the purity was 99.9%.

### Comparative Example 1

To a toluene layer (406 g) of a sulfide body produced in the same method as in Example 1 was added ethanol (20 ml), and reduction with zinc was carried out under the same condition as in Example 1. One hour after completion of dropping of hydrochloric acid, the sulfide body remained in an amount of 8.8%, and even after 90 minutes, remained in an amount of 6.7%.

According to the present invention, bis(3,5-di-tert-butyl-4-hydroxyphenyl) polysulfide can be reduced with zinc in a mixed solvent of toluene and n-butanol to significantly improve stirring efficiency. Further, even if the reaction temperature is controlled around room temperature (15 to 35°C) without heating, the reaction progresses smoothly and 2,6-di-tert-butyl-4-mercaptophenol can be produced efficiently with satisfactory yield, thereby, production cost can be reduced, economically.

## Claims

1. A method for producing 2,6-di-tert-butyl-4-mercaptophenol comprising reducing bis(3,5-di-tert-butyl-4-hydroxyphenyl) polysulfide with zinc in a mixed solvent of toluene and n-butanol.

2. The method according to Claim 1 wherein the reduction is carried out without heating.

3. The method according to Claim 1 wherein the reduction is carried out at a temperature in the range of 15 to 35°C.

4. The method according to Claim 1 wherein the volume ratio of n-butanol to toluene is 5/100 to 30/100.

5. A method for producing 2,6-di-tert-butyl-4-mercaptophenol comprising a process of reacting 2,6-di-tert-butylphenol with sulfur monochloride in toluene to obtain bis(3,5-di-tert-butyl-4-hydroxyphenyl) polysulfide, and a process of reducing bis(3,5-di-tert-butyl-4-hydroxyphenyl) polysulfide with zinc in a mixed solvent of toluene and n-butanol.

6. The method according to Claim 5 wherein the reaction of 2,6-di-tert-butylphenol with sulfur monochloride is carried out at a temperature in the range of -10°C to -7°C.

7. The method according to Claim 5 wherein the reaction in the reduction process is carried out without heating.

8. The method according to Claim 5 wherein the reaction in the reduction process is carried out at a temperature in the range of 15 to 35°C.

9. The method according to Claim 5 wherein the volume ratio of n-butanol to toluene in the reduction process is 5/100 to 30/100.

10. A method for producing 4,4'-isopropylidenedithiobis[2,6-di-tert-butylphenol] comprising a process of reducing bis(3,5-di-tert-butyl-4-hydroxyphenyl) polysulfide with zinc in a mixed solvent of toluene and n-butanol to obtain 2,6-di-tert-butyl-4-mercaptophenol, and a process of reacting 2,6-di-tert-butyl-4-mercaptophenol with acetone under an acidic condition.

11. The method according to Claim 10 wherein the reaction in the reduction process is carried out without heating.

12. The method according to Claim 10 wherein the reaction in the reduction process is carried out at a temperature in the range of 15 to 35°C.

13. The method according to Claim 10 wherein the volume ratio of n-butanol to toluene in the reduction process is 5/100 to 30/100.
